# EUROPEAN PATENT APPLICATION

(11) **EP 1 659 176 A1**
(43) Date of publication of application: **24.05.2006**
(21) Application number: 04720753.5
(22) Date of filing: 15.03.2004
(51) Int. Cl.: C12N 15/12, A61K 31/7105, A61K 31/713, A61K 48/00, A61P 5/24, A61P 5/30, A61P 15/00, A61P 15/08, A61P 15/16, A61P 15/18, A61P 35/00, A61P 35/02, A61P 43/00

(54) **OLIGONUCLEOTIDE INHIBITING THE EXPRESSION OF STAR-BINDING PROTEIN (SBP) GENE AND METHOD THEREFOR**

(30) Priority: 23.07.2003 JP 2003278429
(71) Applicant: Japan Science and Technology Agency, Kawaguchi-shi, Saitama 332-0012 (JP)
(72) Inventor: SUGAWARA, Teruo, Sapporo-shi, Hokkaido 001-0024 (JP)
(74) Representative: Wilson Gunn
(86) International application number: PCT/JP2004/003449
(87) International publication number: WO 2005/010189

(57) **Abstract**

The present invention relates to a means to inhibit the production of StAR binding protein, which binds to StAR protein, i.e. a cholesterol transport stimulating factor, and regulates the function of StAR protein; and furthermore, leads to induce apoptosis specifically in cancer cells by impairing said function. A protein, which interacts with StAR protein, was discovered. Then RNA fragments, which is homologous to a specific nucleotide sequence of said StAR binding protein (SBP), was synthesized and said RNA fragments was transduced into cancer cells. As the results, it was confirmed that the expression of StAR binding protein was inhibited and, furthermore, apoptotic cells emerged.

## Description

### Field of the Invention

The present invention relates to a means to inhibit a production of StAR binding protein, which binds to StAR protein, i.e. a cholesterol transport stimulator, and regulates the function of StAR protein, and more particularly to a means to induce apoptosis specifically in cancer cells by impairing said function.

### Prior Art

StAR protein (acute regulatory protein) plays an important role in transporting cholesterol from mitochondrial outer membrane to inner membrane (Endocr. Rev. 17, 221-224 (1996)). It is thought that StAR protein stimulated the steroid hormone biosynthesis in cytoplasm (Recent Prg Horm Res 54, 396-94 (1999)).

While, apoptosis is a biological process to remove degenerated or transformed cells from a living body. Apoptotic cells are generated in cancer cells by radiation or chemotherapy and have been implicated in cancer treatment; however, said treatment has low specificity to cancer cells and has not been regarded as an effective treatment. Therefore, people thought to induce apoptosis in cancer cells by regulating the function of StAR protein, which is a transport stimulator of cholesterol, i.e. a component of cell membrane (Proc. Natl. Acad. Sci. USA 99, 6943-6948 (2002)).

Also, the present invention used a RNA interference technique to suppress a gene expression by introducing RNA fragments into cells (Nature, 391, 806-811 (1998); WO99/32619).

### Problems to be solved by the Invention

The rate of cancer cell division and growth is higher than those of normal cells and the rate of intracellular metabolism is higher than that of normal cells. Therefore, the present invention focused on the metabolism of cholesterol, which is a cell membrane component, and intend to induce apoptosis specifically in cancer cells by inhibiting the synthesis of a protein, which binds to StAR protein, i.e. a transport stimulator of cholesterol, and regulates the function of StAR protein; and by impairing cellular function.

### Means to solve the Problems

The inventors have been studying the mechanism of stimulation of the steroid hormone biosynthesis in cytoplasm of steroid hormone-producing cells by StAR protein. During the process of the research, the inventors screened proteins, which interact with StAR protein, it was revealed that one of obtained clones contains 2.3 kb inserted gene and interacts with StAR protein. The translation product of the inserted gene was found to be StAR protein binding protein (SBP, DDBJ Accesion number AB112474 (80..2428), AB112475 (431..2779), SEQ ID NO: 1).

The inventors synthesized RNA fragments homologous to the nucleotide sequence of the gene encoding the StAR binding protein and introduced them into cancer cells using a RNA interference technique (Nature, 391, 806-811 (1998); WO99/32619). As the results, it was confirmed that the expression of StAR was inhibited and, furthermore, apoptotic cells emerged.

Namely, the present invention is a sense oligoribonucleotide, an antisense oligoribonucleotide or a double stranded RNA comprised thereof, wherein each oligoribonucleotide comprises less than 23 contiguous nucleotides of the nucleotide sequence of SEQ ID NO: 1 (human StAR binding protein gene) and contains bases 187-205 or 474-494 of SEQ ID NO: 1. The oligoribonucleotide is preferably bases 187-205 or 474-494 of SEQ ID NO: 1.

Moreover, the present invention is a method for inhibiting expression of SBP gene in cancer cells, comprising introducing into cancer cells the sense oligoribonucleotide, the antisense oligoribonucleotide or the double stranded RNA comprised thereof as in above.

Still furthermore, the present invention is a kit for cancer therapy comprising 1) the sense oligoribonucleotide, the antisense oligoribonucleotide or the double stranded RNA comprised thereof as in above and 2) a means to introduce into cancer cells the sense oligoribonucleotide, the antisense oligoribonucleotide or the double stranded RNA comprised thereof.

### Brief Description of the Drawings

Figure 1 shows the result of a pull-down assay of StAR protein and clone 4.
Figure 2 shows the SBP expression in a human tissue.
Figure 3 shows SBP expression in various cell lines. HepG2 indicates human hepatoma cells; KGN indicates human granulosa tumor cells; H295R indicates human adrenal tumor cells; MCF-7 indicates human breast cancer cells.
Figure 4 shows the effect of SBP on steroid biosynthesis. The value shows the increase in the production of pregnenolone by co-introduction of cytochrome P450 scc system and 0.05 µ g SBP.
Figure 5 shows the amount of pregnenolone produced in H295R cells treated with siRNA-SBP-I and siRNA-SBP-II.
Figure 6 shows the amount of pregnenolone produced in KGN cells treated with siRNA-SBP-I and siRNA-SBP-II.
Figure 7 shows the emergence of apoptotic cells by introduction of siRNA (SBPII). Figure 7A shows the result of introduction of siRNA-Scramble; Figure 7B shows the result of introduction of siRNA-SBPII; Figure 7C shows the result of the treatment of those of Figure 7B with DNAase.

### Detailed Description of the Invention

RNA fragments used in the present invention may be sense or antisense RNA to a target RNA, however, said RNA are easily decomposed by RNase and are not effective enough to suppress. Therefore, double strand RNA comprising these RNA is preferably used. The double strand RNA is usually prepared by synthesizing a sense RNA and an antisense RNA separately and then hybridizing these.

The length of said RNA fragments is considered effective at 21~23 nucleotides, however generally a fragment comprising 21 nucleotides is preferably used and, in the examples described later, a fragment comprising 21 nucleotides is actually functional.

The target cells in the present invention are human cancer cells.

The oligoribonucleotide "substantially identical to" a specific nucleotide sequence of SBP gene means the specific part of mRNA generated by the transcription of the gene, which corresponds to the specific nucleotide sequence of SBP gene. That is, more specifically, the ribonucleotide sequence, wherein T in the specific nucleotide sequence of said SBP gene is replaced to U.

There are no restrictions on the method of introducing a RNA fragment into cells and the method includes a method by calcium phosphate, a microinjection method, a protoplast fusion method, electroporation and a method using a virus vector. A commercially available transfection agents comprising liposome etc. are conveniently used.

siRNA of the present invention and the method of using this could be applied to anti-cancer agents and anti-malignant tumor agents for solid tumor: epithelial carcinoma (stomach cancer, lung cancer, hepatic carcinoma, pancreatic cancer etc.), non-solid tumor: leukemia, malignant lymphoma, malignant sarcoma (osteosarcoma, fiblosarcoma etc.), hormone therapy for steroid hormone -dependent malignant tumor (breast cancer, endometrial carcinoma, prostate cancer, ovary cancer etc.), therapeutic products for estrogen-dependent benign disorder (endometriosis, uterine myoma etc.), enhancement and inhibition of sperm maturation, ovulation inducer, contraceptives, therapy of premature puberty, and therapy of gender identity disorder.

The following examples illustrate this invention, however, it is not intended to limit the scope of the invention. The plasmid and cells used in the Examples are prepared or cultured by the following way.

### Construction of a Plasmid

An EcoRI fragment, prepared by PCR using human StAR cDNA as a template, was inserted into a pACT2 vector (CLONTECH Laboratories, Inc., Palo Alto, CA), containing transcriptional factor GAL 4 activation domain (GAD), and a plasmid was constructed after removal the first 62 amino acids, which is a mitochondrial transport signal. The plasmid expresses a fusion protein (GAL4-N-62-StAR) between a transcriptional factor GAL4 and StAR lacking mitochondrial transport signal (i.e. N-62-StAR).

Similarly, GAL4-StAR mutants (GAL4-R193X, GAL4-Q253X, GAL4-frameshift) were constructed.

Also, a plasmid (pVP16-StAR) was constructed by the insertion of an EcoRI fragment prepared from human N-62-StAR cDNA into a pVP16 vector containing an activation domain (AD) derived from VP16 protein of a simple herpes virus (CLO NTECH Laboratories, Inc.).

Also, a plasmid (pM-SBP) was constructed by the insertion of an EcoRI/BamHI fragment of SBP cDNA into pM vector (CLONTECH Laboratories, Inc.) containing GAL4 DNA binding domain (DNA-BD).

Also, a plasmid (pVP16-SBP) was constructed by the insertion of an EcoRI/BamHI fragment of SBP into pVP16 vector (CLONTECH Laboratories, Inc.,). For the preparation of pM-StAR plasmid, an EcoRI fragment of N-62-StAR was cloned and was inserted into pM vector (CLONTECH Laboratories, Inc).

PG5luc (Promega Corp., Madison, WI) contains CAT gene or Luciferase gene as a reporter. An expression plasmid (pSBP) was obtained by the insertion of an EcoRI fragment of the whole coding region amplified by PCR from testis cDNA as a result of RACE into pTarget vector (Promega. Corp.).

These plasmids were transfected by the use of Qiagen Maxiprep system (Qiagen, Hilden, Germany).

### Cell culture

COS-1 cells and human G2 cells are obtained from Riken Cell Bank. Human adrenal cortex tumor cells H295R were gifted from Dr. Okamoto (Osaka University). Human MCF-7 breast cancer cells were obtained from ATCC (Manassas, VA). Human granulosa tumor cells KGN was gifted from Dr. Nishi (Kyushu University).

COS-1 cells were grown on 35 mm plastic dish in DMEM medium supplemented with 10% faetal calf serum and 50 µ g/ml Gentamycin.

KGN cells were cultured in DMEM/F12 medium supplemented with 10% faetal calf serum and 50 µ g/ml Gentamycin.

H295R cells were cultured in DMEM/F12 medium containing 2% ULTROSER G (BioSepra, Cergy-Pontoise, France) and 1% ITS Premix (Becton Dickinson and Co., Franklin Lakes, NJ).

### Example 1

In this invention, the protein interacting with StAR protein was identified by the use of GAL4-based yeast two-hybrid system.

Yeast two-hybrid interaction screening was performed as follows;

pCAT2 plasmid containing human testis cDNA and a reporter gene, which expresses a fusion protein (GAL4-N-62-StAR) between a transcriptional factor GAL4 and StAR (i.e., N-62-StAR) lacking a mitochondrial transport signal, were transduced into a yeast strain CG-1945 (MAT α, ura3-52, his3-200, Iys2-8O1, ade2-101, trpl-901, Ieu2-3, I12, gal4-542, gal80-538, cyh^{r}2. LYS2::GAL1_{UAS}-GAL1_{TATA}-HIS3, URA 3: :_{GAL417mers(x3)}-CyC1_{TATA-}lacZ) (MATCHMAKER Two-Hybrid System 2, C LONTECH). Tansformants (1 x 10⁶) were cultured in a selective synthetic medium (SD) without histidine, leucine and tryptophan at 30°C for 5 days. Whole yeast DNA was transduced into E. coli HB101 by an electroporation. The transduced E. coli cells were cultured and selected in M9 medium without leucine, and then, plasmid DNA was isolated from whole His+ and LacZ+ clones.

Nine clones were obtained. These clones were classified into 3 groups based on DNA sequencing and data based analysis. As shown in Table 1, they are α-helixed coiled-coil rod homolog (HCR, accession NM019052), rabaptin-5 (RAB5EP, accession NM 004703), and nucle obindin 2 (NUCB2, accession NM 005013). HEC and NUCB2 express LacZ phenotype. The clone 4 was selected and analyzed based on the cytoplasmic localization by the prediction of the protein encoded in these clones.

As the results, the clone 4 was found to contain 2.3 kb inserted gene encoding an estimated cytoplasmic protein, which includes 1972 nt open reading frame encoding a protein comprising 657 amino acids and 62 nt non-translated sequence at the 3' end.

**Table 1**

| Clone | Insert size (kb) | Identity | Colony color |
|---|---|---|---|
| 1 | - | - | |
| 4 | 2.3 | HCR | positive |
| 5 | 2.2 | HCR | positive |
| 11 | 3.8 | RAB5EP | negative |
| 26 | 2.6 | NUCB2 | positive |
| 36 | 0.8 | HCR | negative |
| 44 | - | - | |
| 45 | 4.3 | RAB5EP | negative |
| 49 | 2 | HCR | positive |

### Example 2

In this example, a plasmid expressing GAD-clone 4 fusion protein and a plasmid expressing a fusion protein between GAL4-N-62-StAR and GAL4-StAR mutant was examined for the information on the interaction of StAR protein with the clone 4 protein *in vivo.*

A yeast strain Y187 (CLONTECH Laboratories, Inc.) comprising the genotype of MATα, ura3-52, his3-200, ade2-101, trpl-901, leu2-3, 112, ga14Δ, met-, ga180Δ, URA3::_{gal417-mers(x3)}-CyC1_{TATA}-lacZ was used. The Y187 strain was transformed with a fusion plasmid construct between GAL4 and GAD. The transformants are cultured in a selection medium (SD) without leucine and tryptophan at 30°C for 5 days. β-galactosidase activity was determined using x-gal filter assay.

Transfection was performed by the use of two kinds of reverse combinations: one reverse combination is the fusion between the clone 4 and GAL4 and the other reverse combination is the fusion between N-62-StAR and GAD. As shown the result in Table 2, the yeast cells transfected with a hybrid vector between StAR and the clone 4 showed LacZ phenotype, however those transfected with a hybrid vector between a StAR mutant and the clone 4 did not show the phenotype. The result shows that N-62-StAR interacts with the clone 4 in yeast cells.

**Table 2**

| DNA-BD | AD | LacZ Phenotype |
|---|---|---|
| GAL4 | GAD | white |
| GAL4-N-62-StAR | GAD-Clone 4 | blue |
| GAL4-Clone4 | GAD-N-62-StAR | weak blue |
| GAL4-R193X | GAD-Clone 4 | white |
| GAL4-Q258X | GAD-Clone 4 | white |
| GAL4-Frameshift | GAD-Clone 4 | white |

### Example 3

In this example, a pull-down assay was performed for the examination of direct interaction of StAR protein with the clone 4. The pull-down assay was performed according to the following steps;

A plasmid expression clone was prepared by the insertion of an EcoRI fragment obtained by PCR into pCI vector (Promega Corp.). Then, a translation protein was prepared *in vitro* by the use of a TNT-binding reticulocyte dissolving system (Promega Corp.) based on T7 RNA polymerase. Afterward, a plasmid expressing a CBD-N-62-StAR fusion protein (lacking 62 amino acid end) with a His tag was prepared by the insertion of a EcoRI fragment, obtained by PCR by the use of StAR cDNA as a template, into pET38b, containing a His tag (Novagen, San Diego, CA) at the C-terminal. CBD is cellulose binding domain sequence, which is characterized by binding specifically to cellulose and fix a fusion protein to an inactive carrier such as cellulose or chitin without a chemical modification.

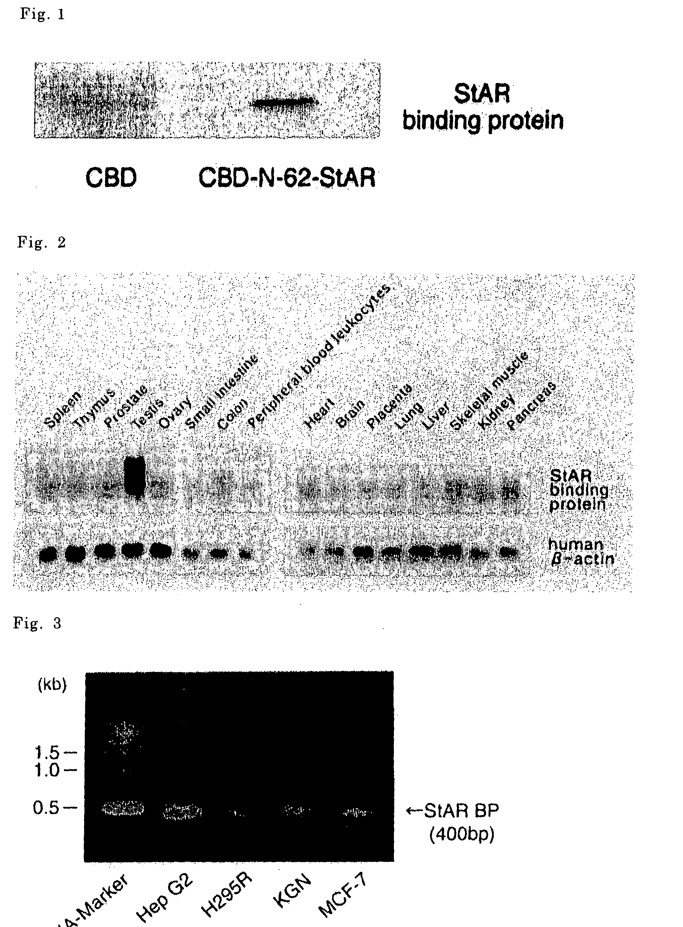

N-62-STAR fusion protein with His tags, bound to His binding resins (Novagen), was incubated in 250 µl buffer solution (50 mM potassium phosphate, pH 7.4, 150 mM KCI, 1 mM MgCl₂, 10% glycerol, 0.1 % Triton-X) together with 50 µl translated clone labeled with ³⁵S methionine for 3 hrs. The resins were collected by a micro-centrifugator and were washed for 3 times. The washed beads were suspended in 20 µl 2xSDS sample buffer solution, heated for 5 min and pelettized. The supernatant was applied to SDS-PAGE and then to autoradiography. Figure 1 shows the result. It is shown that the clone 4 translated protein interacts with CBD-N-62-StAR fusion protein but not with CBD. The clone 4 is referred to as StAR binding protein (SBP) (DDBJ Accession numberAB 112474 (80..2428), AB 112475 (431..2779), SEQ ID NO: 1).

### Example 4

In this example, the expression of SBP was examined by Northern blot analysis.

Northern blot was performed for each 2 µg of poly A and RNA isolated from each tissue by the use of SBP as a probe and β actin cDNA as a positive control. The expression of SBP gene was detected in all tissues examined. Figure 2 shows that the transcription products are abundant in tissues with 2.4 kb or 3.8 kb product.

For the examination of the expression of SBP in various cell lines, RT-PCR was performed by the use of mRNA isolated from HepG2 cells (human hepatic cancer cells), KGN cells (human granulosa tumor cells), H295R cells (human adrenal tumor cells) and MCF-7 cells (human breast cancer cells).

The procedure of RT-PCR was as follows; mRNA used was isolated from Hep G2 cells, KGN cells, H295R cells and MCF-7 cells. Complementary DNA was synthesized by the use of 150 pmol oligodT as a primer, 1 µg total RNA and 200 unit SUPERSCRIPT II Rnase H (Life Technologies, Inc./BRL, Washington, DC) at 37°C for 60 min. The reaction mixture (20 µl) containing reverse transcriptase comprises 50 mM Tris-HCl (pH8.3), 75 mM KCI, 3 mM MgCl₂, 20 mM dithiothreitol and 0.5 mM each of dATP, dCTP, dGTP and dTTP. Then, SBP was amplified by the use of synthesized oligonucleotides of SEQ ID NOs: 2 and 3 as primers. The PCR reaction mixture (50 µl) comprises 10 mM Tris-HCl (pH 8.3), 50 mM KCI, 1.5 mM MgCl₂, 0.2 mM dNTPs and 10 pmol of each primer. PCR reaction condition is 35 cycles, wherein each cycle comprises denaturation at 94°C for 45 sec, the second annealing at 55°C for 45 sec and extension at 72°C for 1 min.

As shown the results in Fig.3, amplified products (400 bp) were obtained from all the cell lines.

The above results show that SBP gene is expressed in steroid hormone-producing cells such as H395R cells and KGN cells.

### Example 5

In this example, the effect of SBP on steroid biosynthesis was examined.

COS-1 cells was cotransfected with F2, cytochrome P450 cholesterol side chain breakage system (Dr. Walter L Miller of the University of California), pStAR (pSPORT StAR CDNA) and pSBP by the use of FuGENE 6. The cells (F2/StAR/SDP) were incubated for 48 hrs after the transfection. Several culture dishes were treated with 22R-hydroxy-cholesterol during the culture of the last 24 hrs. At 48 hrs after the transfection, the medium was recovered and an immunoassay of pregnenolon was performed. The results of the assay were standardized by the concentration of serum pregnenolon, which was produced in the medium containing 22R-hydroxy-cholesterol, to correct the variation of the efficiency of the transfection. Each assay was repeated for three times.

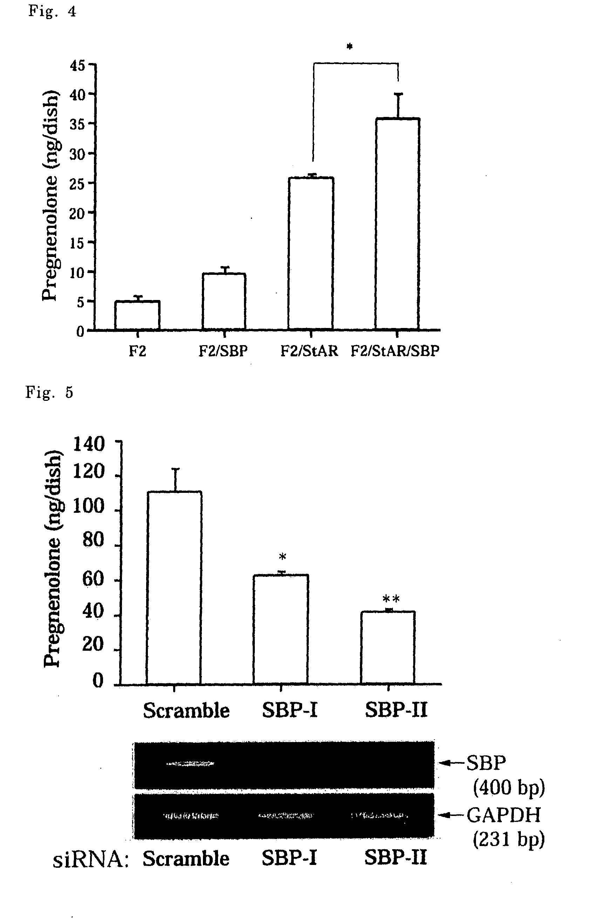

Figure 4 shows the results. The amount of pregnenolone produced by co-transfected COS-1 cells (F2/StAR/SDP) is 138% of that produced in cells (F2/StAR) transfected with F2, StAR or blank vectors.

### Example 6

In this example, SBP expression was suppressed by interference RNA and steroid hormones were examined. Two target sequences in SBP gene was selected and the effect of siRNA was measured by RT-PCR.

The interference RNA assay was performed as follows;

The cultures of subconfluent (40~50% confluent) H295 cells and KGN cells were seeded to 35 mm dishes at similar cell numbers. Nineteen nucleotides double strand RNA (siRNA-SBP-1) and 21 nucleotides double strand RNA (siRNA-SBP-2) were added to said cultures and said cells were transformed to be targeted their endogeneous SBP mRNA (Dharmacon, Inc., Lafayette, Co). The double strand RNAs target 187 and 474 nucleotides downstream from the initiation codon of SBP (SEQ ID NO: 1), respectively.

The siRNAs comprise oligoribonucleotide pairs SBP-1 and SBP-II. SBP-I is 5'-CGGGAUGUUUCCAGUGACAdTdT-3' (SEQ ID NO: 4) and 5'-UGUCACUGGAAACAUCCCGdTdT-3' (SEQ ID NO: 5), SBPII is 5'-GAACUUGGAAGAGGGGAGGCAdTdT-3' (SEQ ID NO: 6) and 5'-UGCCUCCCCUCUUCCAAGUUCdTdT-3' (SEQ ID No: 7). Also, a scramble oligoribonucleotide pair (siRNA-scramble), i.e. 5'-GCGCGCUUUGUAGGAUUCdTdT-3' (SEQ ID NO: 8) and 5'-CGAAUCCUACAAAGCGCGCdTdT-3' (SEQ ID NO: 9), was used as a control.

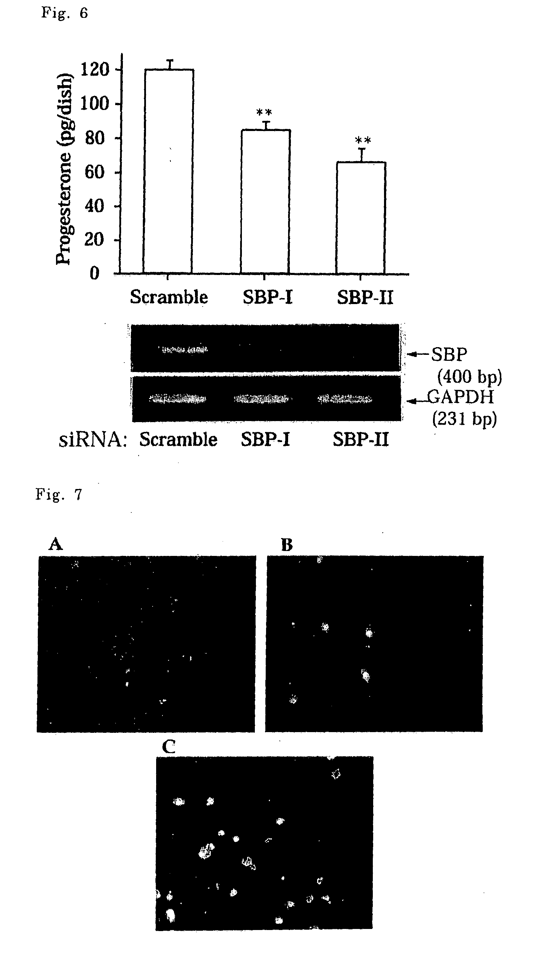

These oligonucleotides were annealed according to a Dharmacon protocol. Cells were transfected with 300 pmol each double strand using 15 µl metafectene (Biontex Laboratories G mbH, Munich, Germany) according to the manufacturer's protocol. The dish of H395R cells was treated with 15 µM triostane (Mochida pharmaceutical co.) for 6 hrs after the transfection for the inhibition of the enzyme activity of 3β-hydroxysteroidhydroxyrase (3β-HSD). 3β-HSD converts pregnenolone into progesterone. At 48 hrs after the transfection, cells were collected and a radioimmunoassay of the steroid hormone was performed. Total RNA was isolated and RT-PCR was performed by the use of primers (sense: SEQ ID NO: 10; anti sense: SEQ ID NO: 11) for GAPDH. The results are shown in Figures 5 and 6.

The amount of pregnenolone produced by H295R cells (85±5.0 ng/dish for those treated with siRNA-SBP-I, 66±8.2 ng/dish for siRNA-SBP-II), treated with siRNA-SBP-I or siRNA-SBP-II after transfected with DsRNA, was 56,5 % or 37.5 %, respectively, of that produced by H295R cells transfected with scramble siRNA, as shown in Fig. 5.

The amount of pregnenolone produced by KGN cells treated with siRNA-SBP-I or siRNA-SBP-II was 71% of 55%, respectively, of that produced by KGN cells transfected with scramble siRNA, as shown in Fig.6.

The expression level of SBP gene was decreased in both siRNA-SBP-I and siRNA-SBP-II cases. These results show that decreased expression of SBP gene by the treatment of the target sequences of SBP gene with siRNA led to the lowered production of the steroid hormone.

### Example 7

In this example, the emergence of apoptotic cells was confirmed by the use of adrenal tumor cells, H295R cells.

H295R cells were cultured in DMEM/F12 medium containing 2% ULTROSER G (BioSepra) and 1 % Premix (Becton Dickinson). The cells were subcultured at a subconfluent state (40-50%) on a 24 mm x 24 mm cover glass placed on a 35 mm plastic culture dish on the day before a gene transfer. For the suppression of the expression of SBP mRNA, the cells were transfected with siRNA-1 or siRNA-II by the use of metafectene. The cultured cells were fixed by phosphate buffered saline containing 4% formaldehyde for 30 min after culturing for 24 hrs after the gene transfer. DeadEnd Fluorometric TUNEL system (Promega) was used for the detection of apoptotic cells and the method was followed a protocol of the system. After two times' wash by phosphate buffered saline, the back of the cover glass was put on a slide glass and observed through a microscope. The observation was performed by the use of a fluorescence microscope (Axiophot, Carl Zeiss) under the condition with 450-490 nm as an excitation wavelength and a 515-565 nm absorption filter. Those cells emitted fluorescent light is apoptotic cells with fractionated DNA. A digital camera (DXM 1200, Nikon) was loaded on a microscope and took the photographs.

The image on the photographs was processed by the use of Adobe Photoshop 5.0 (Adobe system). The magnification of the images is four hundredfold.

Figure 7 (B) shows the result of siRNA-SBP II transfer. Apoptosis cells are recognized entailed by siRNA-SBP-II transfer.

## Claims

1. A sense oligoribonucleotide, an antisense oligoribonucleotide or a double stranded RNA comprising said sense and antisense oligoribonucleotides, wherein the sense oligoribonucleotide is substantially identical to less than 23 contiguous nucleotides of the nucleotide sequence of SEQ ID NO: 1 (human StAR binding protein gene) and contains bases 187-205 or 474-494 of SEQ ID NO: 1.

2. The sense oligoribonucleotide, the antisense oligoribonucleotide or the double stranded RNA comprised thereof as in claim 1, wherein said oligoribonucleotide is bases 187-205 or 474-494 of SEQ ID NO: 1.

3. A method for inhibiting expression of SBP gene in cancer cells, comprising introducing into cancer cells the sense oligoribonucleotide, the antisense oligoribonucleotide or the double stranded RNA comprised thereof as in claim 1 or 2.

4. A kit for cancer therapy comprising 1) the sense oligoribonucleotide, the antisense oligoribonucleotide or the double stranded RNA comprised thereof as in claim 1 or 2 and 2) a means to introduce into cancer cells the sense oligoribonucleotide, the antisense oligoribonucleotide or the double stranded RNA comprised thereof.


SEQUENCE LISTING

<110> Japan Science and Technology Agency

<120> Oligonucleotide Inhibiting The Expression of StAR-Binding Protein (SBP) Gene and Method Therefor

<130> FS04-410PCT

<160> 11

<170> PatentIn version 3.1

<210> 1
<211> 2349
<212> DNA
<213> Homo sapiens

<400> 1
atgtttccac cttcaggttc cactgggctg attcccccct cccactttca agctcggccc 60

ctttcaactc tgccaagaat ggctcccacc tggctctcag acattcccct ggtccaaccc 120

ccaggctatc aagatgtctc agagaggcgg ctagacaccc agagacctca agtgaccatg 180

tgggaacggg atgtttccag tgacaggcag gagccagggc ggagaggcag gtcctggggg 240

ctggaggggt cacaggccct gagccagcag gctgaggtga tcgttcggca gctgcaagag 300

ctgcggcggc tggaggagga ggtccggctc ctgcgggaga cctcgctgca gcagaagatg 360

aggctagagg cccaggccat ggagctagag gctctggcac gggcggagaa ggccggccga 420

gctgaggctg agggcctgcg tgctgctttg gctggggctg aggttgtccg gaagaacttg 480

gaagagggga gccagcggga gctggaagag gttcagaggc tgcaccaaga gcagctgtcc 540

tctttgacac aggctcacga ggaggctctt tccagtttga ccagcaaggc tgagggcttg 600

gagaagtctc tgagtagtct ggaaaccaga agagcagggg aagccaagga gctggccgag 660

gctcagaggg aggccgagct gcttcggaag cagctgagca agacccagga agacttggag 720

gctcaggtga ccctggttga gaatctaaga aaatatgttg gggaacaagt cccttctgag 780

gtccacagcc agacatggga actggagcga cagaagcttc tggaaaccat gcagcacttg 840

caggaggacc gggacagcct gcatgccacc gcggagctgc tgcaggtgcg ggtgcagagc 900

ctcacacaca tcctcgccct gcaggaggag gagctgacca ggaaggttca accttcagat 960

tccctggagc ctgagtttac caggaagtgc cagtccctgc tgaaccgctg gcgggagaag 1020

gtgtttgccc tcatggtgca gctaaaggcc caggagctgg aacacagtga ctctgttaag 1080

cagctgaagg gacaggtggc ctcactccag gaaaaagtga catcccagag ccaggagcag 1140

gccatcctgc agcgatccct gcaggacaaa gccgcagagg tggaggtgga gcgtatgggt 1200

gccaagggcc tgcagttgga gctgagccgt gctcaggagg ccaggcgtca gtggcagcag 1260

cagacagcct cagccgagga gcagctgagg cttgtggtca atgctgtcag cagctctcag 1320

atctggctcg agaccaccat ggctaaggtg gaaggggctg ccgcccagct tcccagcctc 1380

aacaaccgac tcagctatgc tgtccgcaag gtcctcacca ttcggggcct gattgctcga 1440

aagcttgccc ttgctcagct gcgccaggag agctgtcccc taccaccacc ggccacagat 1500

gtgagccttg agttgcagca gctgcgggaa gaacggaacc gcctggatgc agaactgcag 1560

ctgagtgccc gcctcatcca gcaggaggtg ggccgggctc gggagcaagg ggaggcagag 1620

cggcagcagc tgagcaaggt ggcccagcag ctggagcagg agctgcagca gacccaggag 1680

tccctggcta gcttggggct gcagctggag gtagcacgcc agggccagca ggagagcaca 1740

gaggaggctg ccagtctgcg gcaggagctg acccagcagc aggaactcta cgggcaagcc 1800

ctgcaagaaa aggtggctga agtggaaact cggctgcggg agcaactctc agacacagag 1860

aggaggctga acgaggctcg gagggagcat gccaaggccg tggtctcctt acgccagatt 1920

cagcgcagag ccgcccagga aaaggagcgg agccaggaac tcaggcgtct gcaggaggag 1980

gcccggaagg aggaggggca gcgactggcc cggcgcttgc aggagctaga gagggataag 2040

aacctcatgc tggccacctt gcagcaggaa ggtctcctct cccgttacaa gcagcagcga 2100

ctgttgacag ttcttccttc cctactggat aagaagaaat ctgtggtgtc cagccccagg 2160

cctccagagt gttcagcatc tgcacctgta gcagcagcag tgcccaccag ggagtccata 2220

aaagggtccc tctctgtcct gctcgatgac ctgcaggacc tgagtgaagc catttccaaa 2280

gaggaagctg tttgtcaagg agacaacctt gacagatgct ccagctccaa tccccagatg 2340

agcagctaa 2349

<210> 2
<211> 21
<212> DNA
<213> Artificial Sequence

<220>
<223> primer

<400> 2
acttggaggc tcaggtgacc c 21

<210> 3
<211> 20
<212> DNA
<213> Artificial Sequence

<220>
<223> primer

<400> 3
ttcctggagt gaggccacct 20

<210> 4
<211> 19
<212> RNA
<213> Artificial Sequence

<220>
<223> siRNA

<400> 4
cgggauguuu ccagugaca 19

<210> 5
<211> 19
<212> RNA
<213> Artificial Sequence

<220>
<223> siRNA

<400> 5
ugucacugga aacaucccg 19

<210> 6
<211> 21
<212> RNA

<213>  Artificial Sequence

<220>
<223>  siRNA

<400>  6
gaacuuggaa gaggggaggc a                                                21


<210>  7
<211>  21
<212>  RNA
<213>  Artificial Sequence

<220>
<223>  siRNA

<400>  7
ugccuccccu cuuccaaguu c                                                21


<210>  8
<211>  19
<212>  RNA
<213>  Artificial Sequence

<220>
<223>  siRNA

<400>  8
gcgcgcuuug uaggauucg                                                   19


<210>  9
<211>  19
<212>  RNA
<213>  Artificial Sequence

<220>
<223>  siRNA

<400>  9
cgaauccuac aaagcgcgc                                                   19


<210>  10
<211>  20
<212>  DNA
<213>  Artificial Sequence

<220>
<223>  primer

<400>  10
tgccgtctag aaaaacctgc                                                  20


<210>  11
<211>  20
<212>  DNA
<213>  Artificial Sequence

<220>
<223>  primer

<400>  11
accctgttgc tgtagccaaa                                                  20